# EUROPEAN PATENT SPECIFICATION

(11) **EP 2 699 294 B1**
(45) Date of publication and mention of the grant of the patent: **16.03.2016**
(21) Application number: 12715402.9
(22) Date of filing: 20.04.2012
(51) Int. Cl.: A61M 5/315

(54) **PISTON ROD ANCHORING**
KOLBENSTANGENVERANKERUNG
ANCRAGE DE TIGE DE PISTON

(30) Priority: 20.04.2011 EP 11163222; 29.04.2011 US 201161480491 P
(43) Date of publication of application: 26.02.2014
(73) Proprietor: Novo Nordisk Health Care AG, Thurgauerstrasse 36/38 8050 Zürich (CH)
(72) Inventor: MELANDER, Matias, 2880 Bagsværd (DK)
(74) Representative: Vejgaard-Nielsen, Jeppe
(86) International application number: PCT/EP2012/057240
(87) International publication number: WO 2012/143494

(56) References cited:
- EP-A1- 1 892 002
- WO-A1-03/057281
- WO-A1-2010/139793
- US-A1- 2011 034 882

## Description

### FIELD OF THE INVENTION

The present invention relates generally to drug delivery devices and more specifically to mechanisms for coupling a piston driver to a piston in a drug delivery device.

### BACKGROUND OF THE INVENTION

A typical subcutaneous or intravenous drug delivery device comprises a syringe barrel or a cartridge-type reservoir for holding a volume of drug to be delivered, a piston and a piston drive system. The piston drive system comprises a piston rod which interfaces with the piston either directly or through an intermediate coupling element, and which is capable of advancing the piston through the reservoir to expel a dose of the drug.

Some drug delivery devices are applicable for drugs which must be administered to specific compartments in the body. For instance, factor products for the treatment of blood coagulation disorders such as haemophilia are traditionally administered intravenously. When delivering these drugs it is essential to ascertain that the delivery needle is positioned correctly in a vein before the administration is commenced. This may be done after needle insertion by pulling the piston backwards in the reservoir to establish a negative pressure therein, which negative pressure will aspirate a small volume of body fluid in the immediate vicinity of the needle end. From the colour of the body fluid thus entering the reservoir it can be determined whether or not the needle end is in contact with blood.

These drug delivery devices may be adapted for shorter or longer term storage of the drug on either liquid or dry form. In case of storage on dry form, the device may be adapted for reconstitution of the dry drug with a suitable solvent just prior to administration. The solvent may even itself be contained within the particular device along with, but separated from, the dry drug. Exemplary devices offering storage of a drug on dry form as well as reconstitution are the so-called dual chamber devices. In those devices the dry drug is typically arranged in a distal chamber of the reservoir and the solvent is arranged in a proximal chamber of the reservoir. The two chambers are divided by a fluid tight stopper element and the proximal chamber is sealed by a user operable piston. A bypass section may be provided in the reservoir wall for allowing the solvent to pass the stopper element when the stopper element assumes a certain position in the reservoir.

Regardless of whether the drug delivery device holds the drug on liquid form or holds a solvent for reconstitution of a dry drug, the device may during storage and/or handling be exposed to temperature fluctuations which result in an expansion of the present liquid. In such cases it is desirable that the sealing piston is able to displace backwards in the reservoir to allow the liquid to expand. However, if the piston is duly coupled to a piston drive mechanism this drive mechanism will oppose a backward displacement of the piston, in which case the stopper element may displace instead to accommodate the expanding liquid. This is unfortunate since it is desirable to originally position the stopper element close to the bypass section (to minimise the length of the reservoir), and an uncontrolled movement of the stopper element could cause it to actually enter the bypass section, whereby fluid communication would be prematurely established between the two chambers.

It is therefore desirable to provide a drug delivery device wherein in a pre-use state of the device the piston and the piston drive mechanism are uncoupled and spaced apart, and wherein during use or preparation of the device the piston and the piston drive mechanism couple together so as to enable both forward and backward displacements of the piston in the reservoir.

EP 1 892 002 (ACIST Medical Systems, Inc.) discloses a syringe plunger arrangement comprising a pair of spring biased capture members coupled to a distal portion of an actuator. The capture members are adapted to enter into disconnectable engagement with a plunger upon forward movement of the actuator.

In WO 2010/139793 (Novo Nordisk A/S) a piston/piston rod coupling arrangement is disclosed wherein the piston rod has a coupling portion for engagement with a recess in the piston. The coupling portion comprises a number of circumferentially spaced apart segments attached to the piston rod by thin films of material in a manner similar to so-called "living hinges". The segments are structured to protrude radially from a projecting head when in an unloaded state, to pivot about their respective sites of attachment when subjected to a radial force and to return to their initial positions upon termination of the radial force. This provides for a harpoon-like anchoring of the piston rod to the piston once the coupling portion has entered the recess.

### SUMMARY OF THE INVENTION

Such a living hinge attachment solution is attractive because when the piston rod is pressed against the piston the segments fold easily about the piston rod and thus enable virtually effortless entry of the coupling portion into the piston recess. However, the inventor has discovered that following passage through an entrance to the recess which is narrower than the recess itself the returning of the segments to their original positions may be unreliable. Since the returning of the segments to a radially protruding state is crucial in order to provide the desired harpoon effect the intended functionality of the coupling arrangement may in practice be compromised.

In view of the above it is an object of the invention to provide an arrangement for coupling a piston rod and a piston in an easy manner, requiring only an insignificant coupling force.

It is a further object of the invention to provide such an arrangement in a drug delivery device, which arrangement is reliable in the sense that once the piston rod and the piston have been coupled together, they are averse to being decoupled during normal use of the device.

In the disclosure of the present invention, aspects and embodiments will be described which will address one or more of the above objects and/or which will address objects apparent from the below disclosure as well as from the description of exemplary embodiments.

In a first aspect of the invention a piston assembly for a drug delivery device is provided, the piston assembly comprising a piston having a cavity, which cavity comprises a catch portion, a piston rod comprising a coupling member capable of being received in the cavity, and bias means structured to urge a portion of the coupling member into engagement with the catch portion upon entry of the coupling member into the cavity.

The coupling member may comprise a coupling head and a number of (i.e. at least one) deflectable segments or flaps connected thereto at respective connection sites, e.g. via one or more film hinges, and the bias means may be arranged at a distance from the connection sites to thereby provide a separate displacement mechanism which functions independently of the potential elasticity in the connections.

The geometry of the respective deflectable flaps and the bias means may correspond such that when the coupling head is loaded, e.g. radially, the bias means stores energy, and when the load on the coupling head is removed the bias means releases energy, which energy is used to displace the deflectable flaps. This allows for both a reliable and energy efficient construction.

The bias means may be arranged on the piston rod. For example, in some embodiments the bias means is an integral portion of the piston rod, in which case the effect is achieved using a minimum number of parts. In other embodiments the bias means is attached to a portion of the piston rod, whereby a material different from the piston rod material (e.g. a more elastic material) can be used to provide the desired biasing forces. Alternatively, or additionally, the bias means may be arranged in the piston cavity or on a completely separate element.

The bias means may comprise one or more transversally deflectable bias members, e.g. one or more bendable protrusions. The transversally deflectable bias member(s) may extend from the piston rod (either as part(s) of the piston rod or as part(s) of an attachment to the piston rod) in a direction towards the coupling head.

In particular embodiments the piston rod comprises a transversal flange member and the bias means comprises a number of transversally deflectable, e.g. flexible, protrusions extending from the flange member towards the coupling head. In those embodiments the number of protrusions may at least equal the number of deflectable flaps, whereby it can be ensured that each deflectable flap is impacted as desired. The piston rod may further comprise a longitudinal shaft, and the transversal flange member may be arranged on the longitudinal shaft, e.g. as a unitary portion thereof.

Alternatively, or additionally, the bias means may comprise one or more deflectable protrusions extending transversally, at one or more respective angles, from the longitudinal shaft.

The flange member and the protrusions may be arranged such that when a medially, or radially inwardly, directed force is applied to the deflectable flaps to thereby press the deflectable flaps towards the coupling member a portion of the respective deflectable flaps engages a portion of the respective protrusions and bends the protrusions towards the coupling member. In case the protrusions are flexible this will cause an accumulation of energy in each of them.

The piston cavity may comprise a main hollow portion and an entrance section of narrower width than the main hollow portion. In particular, the main hollow portion may comprise a generally conical configuration which opens in a direction towards the entrance section and then tapers off to provide a bottleneck construction in which the tapering portion may serve as a catch or interface portion for the deflectable flaps.

The piston and the piston cavity may be axi-symmetrical, and the coupling member may comprise a reduced diameter portion of the piston rod. The flange member may be arranged at the diameter constriction and the protrusions may extend from the flange at a radial, or transversal, distance from the longitudinal piston rod axis which corresponds, or corresponds substantially, to half the width of the entrance section. Thereby, the protrusions may protrude through the entrance section and into the main hollow portion when the coupling head is inserted into the piston cavity to urge the deflectable flaps into maximum engagement with the catch portion.

Once the coupling head is inserted into the piston cavity and the deflectable flaps engage properly with the catch portion an axial force trying to separate the piston rod from the piston will cause the deflectable flaps to engage more tightly with the catch portion, thereby providing a harpoon-like interface between the piston rod and the piston.

In a second aspect of the invention a drug delivery device is provided which comprises a piston assembly as described in the above. The drug delivery device may e.g. be of the injection pen type or the infusion pump type. In particular embodiments, the drug delivery device is a dual chamber mixing and administration device which further comprises a reservoir having an outlet end portion and an activation end portion, a slidable stopper arranged between the outlet end portion and the activation end portion, and passage means for enabling fluid passage from a first side to a second side of the stopper. The piston is arranged between the stopper and the activation end portion. A first substance may be arranged in the reservoir between the outlet end portion and the stopper, and a second substance may be arranged between the stopper and the piston.

A piston rod drive mechanism may be provided for manual or automatic movement of the piston rod relative to the reservoir, and the piston rod may in a pre-use state be positioned axially spaced apart from the piston to thereby allow unbiased axial motion of the piston in the reservoir.

In particular embodiments an initial operation of the piston rod drive mechanism will cause an advancement of the piston rod towards the piston, whereby the coupling member will be pushed into the cavity and the piston rod anchoring will take place.

In a third aspect of the invention a piston rod for use in a drug delivery device is provided, the piston rod comprising a longitudinal shaft, a coupling member having a transversally variable extent, and bias means for urging the coupling member towards a maximum transversal extent. The coupling member may comprise a coupling head and a number of transversally deflectable segments connected thereto at respective connection sites, and the bias means may be arranged at a distance from these connection sites. The particular configurations and arrangements of the coupling member and the bias means may be any of the herein described.

In a fourth aspect of the invention a method for coupling a piston rod to a piston is provided, the method comprising:
∘ providing a piston comprising a piston body and a cavity, the cavity comprising a narrow entrance portion and a catch portion,
∘ providing a piston rod comprising a longitudinal shaft, a coupling member having a transversally variable dimension and bias means for influencing the transversal dimension of the coupling member, and
∘ inducing relative motion between the piston and the piston rod to thereby force the coupling member to enter the cavity through the entrance portion, during which entry the coupling member firstly contracts and deforms the bias means and secondly expands into alignment or engagement with the catch portion due to an elastic recovery of the bias means.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context.

The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Fig. 1 shows a cross-sectional view of a piston assembly according to an embodiment of the invention, where the piston and the piston rod are seen in a pre-use relative position,
Fig. 2 shows a cross-sectional view of the piston assembly during insertion of the piston rod into the piston cavity, and
Fig. 3 shows a cross-sectional view of the piston assembly in a coupled state.

In the figures like structures are mainly identified by like reference numerals.

### DESCRIPTION OF EXEMPLARY EMBODIMENTS

When in the following relative expressions, such as "forward" and "backward", are used, these refer to the appended figures and not necessarily to an actual situation of use. The shown figures are schematic representations for which reason the configuration of the different structures as well as their relative dimensions are intended to serve illustrative purposes only.

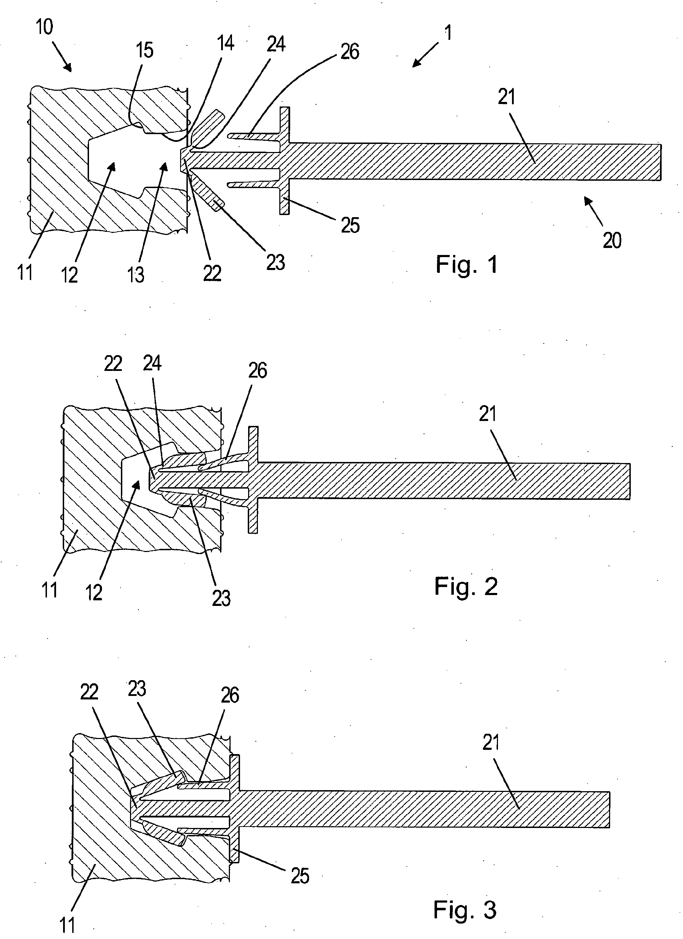

Fig. 1 is a cross-sectional representation of a piston assembly 1 according to an exemplary embodiment of the invention. The piston assembly 1 comprises a piston 10 and a piston rod 20. The piston 10 is arranged sealingly in a cartridge (not shown) and has a body 11, made of e.g. a rubber material, which body 11 is provided with a hollow 12 that is accessible via an entrance 13. An entrance wall portion 14 tapers towards the hollow 12 and widens in the transition between the entrance 13 and the hollow 12 to thereby provide a circumferential barb 15.

The piston rod 20 comprises a longitudinal shaft 21, the distal portion of which constitutes a coupling member having a reduced diameter relative to the diameter of the proximal portion of the shaft 21. At its distal end the coupling member has a head 22 and two opposite flaps 23 connected to the head 22 via respective film hinges 24. The piston rod 20 further has a transversal flange 25 arranged between the large diameter shaft portion and the smaller diameter shaft portion. Two opposite flexible fingers 26 extend from the distal side of the flange 25 towards the head 22.

In the pre-use state of the piston assembly 1 shown in Fig. 1 the piston 10 and the piston rod 20 are capable of relative motion towards as well as away from one another.

Fig. 2 shows the piston assembly 1 in a state where the piston 10 and the piston rod 20 are about to be coupled together, and Fig. 3 shows the piston assembly 1 in a coupled state. These figures thus disclose the functionality of the piston rod anchoring mechanism as it is manifested in accordance with this particular embodiment of the invention.

In Fig. 2 the shaft 21 has been moved towards the body 11, whereby the head 22 has been forced through the entrance 13 and into the hollow 12. The flaps 23 have been forced by radial compressive forces due to the interaction with the entrance wall portion 14 to pivot about the respective film hinges 24 and fold about the reduced diameter piston rod portion. In doing that the proximal most portions of the flaps 23 have engaged the distal most portions of the flexible fingers 26 and as a result the flexible fingers 26 have been bent towards the reduced diameter piston rod portion.

The head 22 can be inserted into the hollow 12 using a very small axial force which practically only has to overcome the flexural rigidity of the fingers 26. The piston rod 20 is e.g. made of a suitable plastic material and the flexible fingers 26 are slender as they rieed only have a relatively small flexural rigidity to urge the flaps 23 away from the reduced diameter piston rod portion once the flaps 23 are completely within the hollow 12.

In Fig. 3 the head 22 has been inserted completely into the hollow 12 and the flaps 23 have been deflected away from the reduced diameter piston rod portion into alignment with a conical wall portion of the hollow 12 and the circumferential barb 15 by the flexible fingers 26, which have elastically returned to their original, or substantially original, positions following the complete passage of the flaps 23 through the entrance 13. In the relative position of the piston 10 and the piston rod 20 shown in Fig. 3 the two elements are only capable of joint motion. Due to the harpoon-like interface between the flaps 23 and the circumferential barb 15 the joint motion can be carried out in both a forward and backward direction, thereby enabling both distal and proximal displacement of the piston 10 in the cartridge.

## Claims

1. A piston assembly (1) for a drug delivery device, the piston assembly (1) comprising:
- a piston (10) having a cavity (12), which cavity (12) comprises a catch portion (15),
- a piston rod (20) comprising a longitudinal shaft (21) and a coupling member capable of reception in the cavity (12), the coupling member comprising a coupling head (22) and a number of deflectable flaps (23) connected thereto at respective connection sites (24), and
- bias means structured to urge a portion of the respective deflectable flaps (23) into alignment or engagement with the catch portion (15) upon entry of the coupling member into the cavity (12),
**characterised in that** the bias means comprises a number of transversally deflectable bias members (26) extending from the piston rod (20) in a direction towards the coupling head (22).

2. An assembly according to claim 1, wherein the piston rod (20) further comprises a flange member (25), and wherein the bias members (26) extend from the flange member (25).

3. An assembly according to claim 1 or 2, wherein the bias members (26) are arranged such that when a medially directed force presses the deflectable flaps (23) towards the coupling head (22) a portion of the respective deflectable flaps (23) engages a portion of the respective bias members (26) and bends the bias members (26), thereby accumulating energy therein.

4. An assembly according to claim 3, wherein when the medially directed force is discontinued the accumulated energy in the bias members (26) is released, whereby the respective bias members (26) urge the respective deflectable flaps (23) away from the coupling head (22).

5. An assembly according to any of the preceding claims, wherein the cavity (12) comprises an entrance portion (13) and a main portion, the smallest transversal dimension of the entrance portion (13) being smaller than the largest transversal dimension of the main portion, and wherein the catch portion (15) is constituted by a tapering from the largest transversal dimension of the main portion to the smallest transversal dimension of the entrance portion (13).

6. An assembly according to claim 5, when dependent on any of claims 2 - 4, wherein each bias member (26) extends from the flange member (25) at a transversal distance from a central axis of the longitudinal shaft (21) which corresponds substantially to half the smallest transversal dimension of the entrance portion (13).

7. A drug delivery device comprising a piston assembly (1) according to any of claims 1 - 6.

8. A piston rod (20) for use in a drug delivery device, the piston rod (20) comprising a longitudinal shaft (21), a coupling member having a transversally variable extent and a number of protrusions (26) adapted to urge the coupling member towards a maximum transversal extent.

9. A piston rod according to claim 8, wherein the coupling member comprises a number of transversally deflectable flaps (23). I

## Patentansprüche

1. Kolbenanordnung (1) für eine Arzneimittelverabreichungsvorrichtung, wobei die Kolbenanordnung (1) Folgendes umfasst:
- einen Kolben (10) mit einem Hohlraum (12), der einen Rastabschnitt (15) umfasst,
- eine Kolbenstange (20), die einen Längsschaft (21) und ein im Hohlraum (12) aufnehmbares Koppelelement umfasst, wobei das Koppelelement einen Koppelkopf (22) und mehrere umlenkbare Laschen (23) umfasst, die an jeweiligen Verbindungsstellen (24) damit verbunden sind, und
- ein Vorspannmittel, das so strukturiert ist, dass es einen Abschnitt der jeweiligen umlenkbaren Laschen (23) in Ausrichtung auf den oder in Eingriff mit dem Rastabschnitt (15) drängt, wenn das Koppelelement in den Hohlraum (12) eintritt,
**dadurch gekennzeichnet, dass** das Vorspannmittel mehrere quer umlenkbare Vorspannelemente (26) umfasst, die sich von der Kolbenstange (20) in eine zum Koppelkopf (22) hin gehende Richtung erstrecken.

2. Anordnung nach Anspruch 1, wobei die Kolbenstange (20) ferner ein Flanschelement (25) umfasst und wobei sich die Vorspannelemente (26) von dem Flanschelement (25) erstrecken.

3. Anordnung nach Anspruch 1 oder 2, wobei die Vorspannelemente (26) so angeordnet sind, dass ein Abschnitt der jeweiligen umlenkbaren Laschen (23), wenn eine medial gerichtete Kraft die umlenkbaren Laschen (23) zum Koppelkopf (22) hin drückt, einen Abschnitt der jeweiligen Vorspannelemente (26) in Eingriff nimmt und die Vorspannelemente (26) biegt, wodurch darin Energie gespeichert wird.

4. Anordnung nach Anspruch 3, wobei die in den Vorspannelementen (26) gespeicherte Energie freigesetzt wird, wenn die medial gerichtete Kraft ausgesetzt wird, wodurch die jeweiligen Vorspannelemente (26) die jeweiligen umlenkbaren Laschen (23) vom Koppelkopf (22) weg drängen.

5. Anordnung nach einem der vorhergehenden Ansprüche, wobei der Hohlraum (12) einen Eingangsabschnitt (13) und einen Hauptabschnitt umfassst, wobei die kleinste Querabmessung des Eingangsabschnitts (13) kleiner als die größte Querabmessung des Hauptabschnitts ist, und wobei der Rastabschnitt (15) aus einer Verjüngung von der größten Querabmessung des Hauptabschnitts auf die kleinste Querabmessung des Eingangsabschnitts (13) besteht.

6. Anordnung nach Anspruch 5, wenn von einem der Ansprüche 2 - 4 abhängig, wobei sich jedes Vorspannelement (26) in einem im Wesentlichen der Hälfte der kleinsten Querabmessung des Eingangsabschnitts (13) entsprechenden Querabstand von einer mittleren Achse des Längsschafts (21) vom Flanschelement (25) erstreckt.

7. Arzneimittelverabreichungsvorrichtung, umfassend eine Kolbenanordnung (1) nach einem der Ansprüche 1 - 6.

8. Kolbenstange (20) zur Verwendung in einer Arzneimittelverabreichungsvorrichtung, wobei die Kolbenstange (20) einen Längsschaft (21), ein Koppelelement mit einer quervariablen Erstreckung und mehrere Vorsprünge (26) umfasst, die geeignet sind, das Koppelelement zu einer maximalen Quererstreckung zu drängen.

9. Kolbenstange nach Anspruch 8, wobei das Koppelelement mehrere quer umlenkbare Laschen (23) umfasst.

## Revendications

1. Ensemble piston (1) pour un dispositif d'administration de médicament, l'ensemble piston (1) comprenant :
- un piston (10) comportant une cavité (12), ladite cavité (12) comprenant une partie de blocage (15),
- une tige de piston (20) comprenant une broche longitudinale (21) et un élément d'accouplement pouvant être reçu dans la cavité (12), l'élément d'accouplement comprenant une tête d'accouplement (22) et un certain nombre de languettes fléchissables (23) raccordées à celle-ci au niveau de points de raccordement (24) respectifs, et
- des moyens de sollicitation structurés de façon à amener une partie des languettes fléchissables (23) respectives en alignement ou en prise avec la partie de blocage (15) lors de la pénétration de l'élément d'accouplement dans la cavité (12),
**caractérisé en ce que** les moyens de sollicitation comprennent un certain nombre d'éléments de sollicitation fléchissables transversalement (26) s'étendant à partir de la tige de piston (20) en direction de la tête d'accouplement (22).

2. Ensemble selon la revendication 1, dans lequel la tige de piston (20) comprend en outre un élément formant collerette (25), et dans lequel les éléments de sollicitation (26) s'étendent à partir de l'élément formant collerette (25).

3. Ensemble selon la revendication 1 ou 2, dans lequel les éléments de sollicitation (26) sont conçus de telle sorte que, lorsqu'une force s'exerçant dans une direction médiale applique une pression sur les languettes fléchissables (23) en direction de la tête d'accouplement (22), une partie des languettes fléchissables (23) respectives vient en contact avec une partie des éléments de sollicitation (26) respectifs et courbe les éléments de sollicitation (26), de sorte que de l'énergie s'accumule dans ceux-ci.

4. Ensemble selon la revendication 3, dans lequel, lorsque la force s'exerçant dans une direction médiale est interrompue, l'énergie accumulée dans les éléments de sollicitation (26) est libérée, les éléments de sollicitation (26) respectifs poussant alors les languettes fléchissables (23) respectives de façon à les écarter de la tête d'accouplement (22).

5. Ensemble selon l'une quelconque des revendications précédentes, dans lequel la cavité (12) comprend une partie d'entrée (13) et une partie principale, la dimension transversale la plus petite de la partie d'entrée (13) étant plus petite que la dimension transversale la plus grande de la partie principale, et dans lequel la partie de blocage (15) est formée par un effilement de la dimension transversale la plus grande de la partie principale à la dimension transversale la plus petite de la partie d'entrée (13).

6. Ensemble selon la revendication 5, lorsqu'elle dépend de l'une quelconque des revendications 2 à 4, dans lequel chaque élément de sollicitation (26) s'étend à partir de l'élément formant collerette (25) à une distance transversale vis-à-vis d'un axe central de la broche longitudinale (21) qui correspond essentiellement à la moitié de la dimension transversale la plus petite de la partie d'entrée (13).

7. Dispositif d'administration de médicament comprenant un ensemble piston (1) selon l'une quelconque des revendications 1 à 6.

8. Tige de piston (20) destinée à être utilisée dans un dispositif d'administration de médicament, la tige de piston (20) comprenant une broche longitudinale (21), un élément d'accouplement présentant une étendue variable dans le sens transversal et un certain nombre de saillies (26) conçues pour pousser l'élément d'accouplement vers une étendue transversale maximale.

9. Tige de piston selon la revendication 8, dans laquelle l'élément d'accouplement comprend un certain nombre de languettes fléchissables transversalement (23).
